# EUROPEAN PATENT SPECIFICATION

(11) **EP 2 181 719 B1**
(45) Date of publication and mention of the grant of the patent: **23.11.2011**
(21) Application number: 09169152.7
(22) Date of filing: 01.09.2009
(51) Int. Cl.: A61L 2/26, A61L 2/28, A61B 19/02

(54) **Self-sealed medical sterilization pouch**
Selbstabgedichteter medizinischer Sterilisationsbeutel
Poche de stérilisation médicale auto-étanche

(30) Priority: 31.10.2008 TW 97219503 U
(43) Date of publication of application: 05.05.2010
(73) Proprietor: Sigma Medical Supplies Corporation, Jui-Fang Chen (TW)
(72) Inventor: Kuo, Chun-Cheng, Taipei County (TW); Chou, Chen-Tan, Taipei County (TW)
(74) Representative: Becker Kurig Straus

(56) References cited:
- WO-A-2009/091673
- US-A- 3 627 611
- US-A- 4 091 921
- US-A- 5 344 017

## Description

### BACKGROUND OF THE INVENTION

### 1. Field of Invention

The invention relates to a self-sealed medical sterilization pouch, more particularly to a self-sealed medical instrument sterilization pouch for being pervious to steam, water vapor and sterilization gases in order to prevent the contamination from ink.

### 2. Description of Related Art

Medical instruments have to be gathered after use, cleaned, packaged in sterilizable pouches. Medical instruments are sealed in a closed space defined by the pouch walls, and then subjected to a sterilizing environment such as steam or high temperature water vapor used to sterilize the instruments. Besides, the pouch-enclosed instruments are subjected to a sterilizing gas such as ethylene oxide.

The sealed pouches comprise a lower sheet and an upper sheet. The lower sheet is typically planar sheet material, suitably cellulosic in composition and printable, and pervious to steam, water vapor and sterilization gases. The upper sheet is planar, but typically of transparent plastic such as polyester, impervious to water vapor, steam and sterilizing gases. Moreover, to make sure that a given pouch has been sterilized, indicators are used. One way to make sure that a given pouch has been sterilized is to provide a separate indictor-strip into the pouch. Besides, the indicators typically are printing inks having color change response to steam, high temperature water vapor or a sterilizing gas such as ethylene oxide, or other sterilizing agent. For example, the ink permanently changes from gray to brown when exposed to sterilizing conditions.

Conventional sterilizing pouches are formed of sheet material printed with identifying brand names and other information on an outward face, and the indictor ink has been placed on the same face. In addition, in order to protect ink dot from deleterious frictional contact with the medical instruments, a protective barrier is preferably provided between the dot and the medical instruments. The barrier is integrated into a seal line by bifurcating the seal line at the apex thereof to form a diamond-shaped enclosure within which the indictor ink dot is placed.

U.S. Patent No. 4,091,921A discloses a sterilizable package which has a plastic member marginally heat sealed to a paper member to which is applied indicia that changes color upon sterilization (gas and/or steam) on the inside of the package. To prevent the indicia from being covered by the package contents, and to avoid contamination of the package contents by the indicia, the area surrounding the indicia is heat sealed.

U.S. Patent No. 5,344,017A discloses an instrument pouch with in-pouch sterile processing indicator, the pouch has first and second sheet material walls which are joined to define the pouch, and the joinder of the walls defines a perimetrical seal about a partially closed volume within which instruments are placed for sterilization. The pouch also has a flap on one of the walls arranged to overlie the other of the walls in sealed relation to fully enclose the volume. A sterile processing indicator is within the enclosed volume and adjacent the perimetrical seal, and there is further included means blocking frictional contact between the sterile processing indicator and the instruments within the enclosed volume.

U.S. Patent No. 3,627,611A discloses a method and an apparatus for the manufacture of surgical pouches. A pouch is comprised of a front, a header and a backer, and has an insertion opening at one end and a removal opening at the other end. In a finished pouch, the removal opening area is closed by a chevron seal, and an instrument to be packaged may be inserted into the pouch through insertion opening which would then be heat-sealed shut.

However, the conventional sterilizing pouches have several drawbacks. First, the conventional sterilizing pouches are lack of protection for the ink from frictional removal during handling and storage. Medical instruments may be contaminated by ink. Second, the separate indictor-strip is easy to be polluted. Finally, the conventional sterilizing pouches have a V-shaped seal line to form the barrier, but the seal line is easy to be breached by the medical instruments during handling and storage. Thus, the need for improvement still exists.

### SUMMARY OF THE INVENTION

One objective of the present invention is to provide a self-sealed medical sterilization pouch, wherein the medical instruments are able to keep clean and prevent the contamination from ink.

Another objective of the present invention is to provide a self-sealed medical sterilization pouch, wherein the indictors are protected by seal lines to prevent the pollutions.

A further objective of the present invention is to provide a self-sealed medical sterilization pouch, wherein the seal lines are not easy to be breached by the medical instruments during handling and storage.

The self-sealed medical sterilization pouch comprises the following elements of: a first sheet; a second sheet joined with the first sheet by a first seal line, a second seal line, and a third seal line on the edge of the first sheet and the second sheet to form a closed space with an opening, and the closed space can accommodate at least one medical instrument to be sterilized, wherein the third seal line being the most internal of the three seal lines; at least one printing domain provided on the surface of the first sheet and blocked by the first seal line and the second seal line to keep the closed space clean; a flap provided on one end of the first sheet and located on the side the opening is toward; a folding line being between the closed space and the flap; a separating seal line being provided in the closed space and being close to the third sealed line, the separating seal line comprising an arched portion, a first ladle-shaped end, and a second ladle-shaped end, wherein the first ladle-shaped end and the second ladle-shaped end connected to two ends of the arched portion respectively; and at least two sterile processing indicators provided on the surface of the first sheet and disposed between the third seal line and the first ladle-shaped end and the third seal line and the second ladle-shaped end respectively.

The invention will become more obvious from the following description when taken in connection with the accompanying drawings.

### BRIEF DESCRIPTION OF THE DRAWINGS

Fig. 1 is an exploded view of a preferred embodiment of a self-sealed medical sterilization pouch according to the invention;
Fig. 2 is a perspective view of the self-sealed medical sterilization pouch according to the invention;
Fig. 3 is a view of the self-sealed medical sterilization pouch containing medical instruments inside according to the invention; and
Fig. 4 is a view of how to open the self-sealed medical sterilization pouch containing medical instruments inside according to the invention.

### DETAILED DESCRIPTION OF THE INVENTION

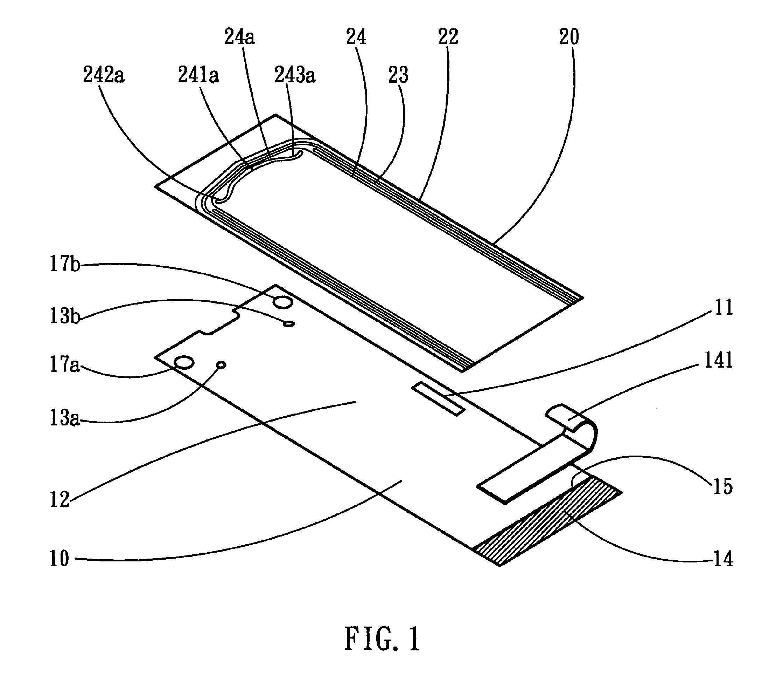

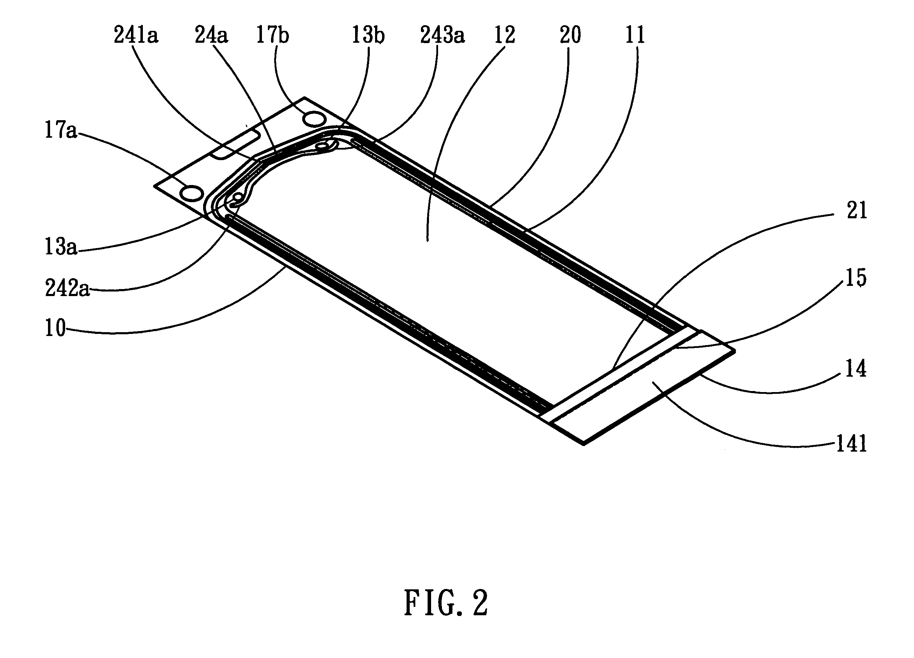

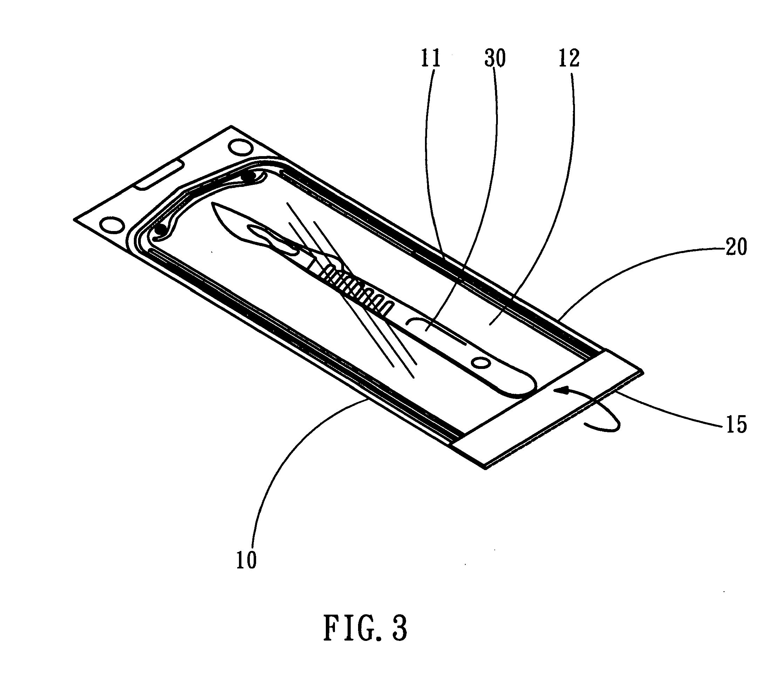

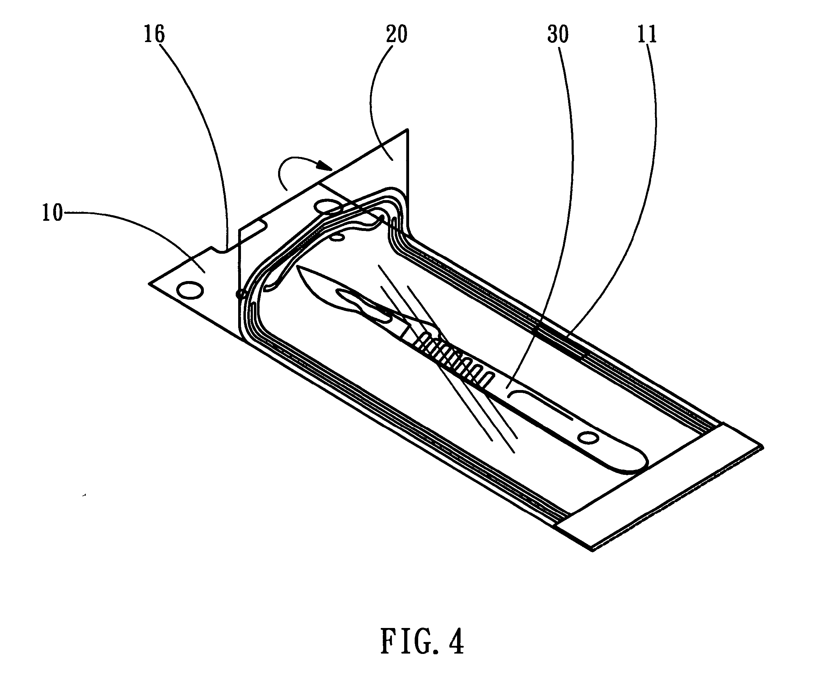

Referring to Figs. 1 to 4, a self-sealed medical sterilization pouch in accordance with a preferred embodiment of the invention is shown. The present invention is to provide the self-sealed medical sterilization pouch and includes: a first sheet 10, which is pervious to steam, water vapor and sterilization gases; a second sheet 20, which is impervious to steam, water vapor and sterilization gases, and the second sheet 20 joined with the first sheet 10 by a first seal line 22, a second seal line 23, and a third seal line 24 on the edge of the first sheet 10 and the second sheet 20 to form a closed space 12 with an opening 21, wherein the closed space 12 can accommodate at least one medical instrument 30 to be sterilized, and wherein the third seal line 24 is the most internal of the three seal lines; at least one printing domain 11 being provided on the surface of the first sheet 10 and blocked by the first seal line 22 and the second seal line 23 to keep the closed space 12 clean, and a plurality of signs as words or other symbols being disposed on the printing domain 11; a flap 14 being provided on one end of the first sheet 10 and located on the side the opening 21; a folding line 15 being between the closed space 12 and the flap 14; a separating seal line 24a being provided in the closed space 12 and close to the third sealed line 24, wherein the separating seal line 24a comprises an arched portion 241a, a first ladle-shaped end 242a, and a second ladle-shaped end 243a, the first ladle-shaped end 242a and the second ladle-shaped end 243a being connected to two ends of the arched portion 241a respectively; and at least two sterile processing indicators 13a, 13b being provided on the surface of the first sheet 10 and disposed between the third seal line 24 and the first ladle-shaped end 242a and the third seal line 24 and the second ladle-shaped end 243a respectively.

The second sheet 20 is made of non-tearing film. It is a superior film to have complete film separation at any speed or condition opening, without shearing and tearing, to maintain clean-opening aseptically. Thus, the pouch utilizes protecting seal at chevron side to prevent packed item's breaching by the medical instrument 30. The third sealed line 24 can effectively prevent the medical instrument 30 contacting ink.

The flap 14 on the second sheet 20 is arranged to overlie the first sheet 10 in seal for fully enclosing the closed space 12, wherein the two sterile processing indicators 13 are adjacent to the second seal line 23 and the third seal line 24, the two sterile processing indicator 13a, 13b are surrounded by the second seal line 23 and the first ladle-shaped end 242a and second ladle-shaped end 243a respectively. The indicator 13a, 13b can fulfill pouch-inside sterilant contacting and self-sealed pouch, additionally, can save on putting separate indicator-strip into pouch and free of polluting. The flap 14 includes a protecting paper 141 and the flap 14 is adhesive to the second sheet 20 while the protecting paper 141 is tore off and the folding line 15 is folded up, so that the closed space 12 is completely sealed.

Preferably, the sterilization gases can be ethylene oxide, hydrogen peroxide gas, inorganic agents, organic agents suitable, etc. for such purposes.

Preferably, the second sheet 20 is made of non-tearing film or plastic.

Preferably, the two sterile processing indicators 13a, 13b are sterile ink dots and separately responsive to different sterilizing agents. The inks used to print the sterile ink dots are conventional in the industry for monitoring sterilization exposure to various sterilants.

Preferably, the second sheet 20 includes a gap 16 formed at one end of the second sheet 20. The gap 16 makes the separation of the first sheet 10 and the second sheet 20 easily.

Moreover, the preferred embodiment of the invention can be provided two compared indicators 17a, 17b. The two compared indicators 17a, 17b are located on the surface of the first sheet 10 and exterior of the closed space 12. One of the sterile processing indicators 13a and one of the compared indicators 17a are responsive to multi-parameter steam sterilization, the other sterile processing indicators 13b and the other compared indicators 17b are responsive to gas (such as ethylene oxide) sterilization.

Through the invention, the following advantages are obtained. First, the invention provides a self-sealed medical sterilization pouch. The separating sealed line comprises an arched portion and two ladle-shaped ends. Thus, the pouch utilizes protecting seal at chevron side to prevent packed item's breaching by medical instruments. The third sealed line can effectively prevent the medical instruments from contacting the ink.

Second, the second sheet is made of non-tearing film. It is a superior film to have complete film separation at any speed or condition opening, without shearing and tearing, to maintain clean-opening aseptically.

Third, the first sheet is made of first grade medical paper. The medical grade paper provides optimal barrier properties and offers higher level security.

Fourth, the present invention utilizes printed-in internal indicator. The indicator can fulfill pouch-inside sterilant contacting and self-sealed pouches, additionally, can save on putting separate indicator-strip into pouch and free of polluting.

Fifth, in order to assure sterility sterilization, packed items should be sterilized to three sterilization parameters: time, temperature and sterilant contact. The present invention is followed by ISO standards and CDC infection control guidelines. Finally, free-polluted printing is used. The present invention uses water-based ink and printed outside actual packing area. It can be free of polluting on packed items.

While the invention herein disclosed has been described by means of specific embodiments, numerous modifications and variations could be made thereto by those skilled in the art without departing from the scope of the invention set forth in the claims.

## Claims

1. A self-sealed medical sterilization pouch having a first sheet (10), a second sheet (20) being joined with the first sheet (10) by a first seal line (22), a second seal line (23), and a third seal line (24) which are disposed on the edge of three laterals of the first sheet (10) and the second sheet (20) and to form a closed space (12) with an opening (21), and the closed space (12) accommodating at least one medical instrument (30) to be sterilized, wherein the third seal line (24) is the most internal of the three seal lines (22, 23 and 24),
at least one printing domain (11) being provided on the surface of the first sheet (10) and blocked by the first seal line (22) and the second seal line (23) to keep the closed space (12) clean,
a flap (14) being provided on one end of the first sheet (10) and located on the side the opening (21), a folding line (15) being between the closed space (12) and the flap (14),
a separating seal line (24a) being provided in the closed space (12) and close to the third sealed line (24),
and at least two sterile processing indicators (13a and 13b) being provided on the surface of the first sheet (10), wherein
the separating seal line (24a) comprising an arched portion (241a), a first ladle-shaped end (242a), and a second ladle-shaped end (243a), wherein the first ladle-shaped end (242a) and the second ladle-shaped end (243a) are connected to two ends of the arched portion (241a) respectively; and
the at least two sterile processing indicators (13a and 13b) being disposed between the third seal line (24) and the first ladle-shaped end (242a) and between the third seal line (24) and the second ladle-shaped end (243a) respectively.

2. The self-sealed medical sterilization pouch of claim 1, wherein the first sheet (10) being pervious to steam, water vapor and a sterilization gas.

3. The self-sealed medical sterilization pouch of claim 2, wherein the sterilization gas is selected from a group consisting of: ethylene oxide, hydrogen peroxide gas, inorganic agents, and organic agents.

4. The self-sealed medical sterilization pouch of claim 1, wherein the second sheet (20) being impervious to steam, water vapor and a sterilization gas.

5. The self-sealed medical sterilization pouch of claim 4, wherein the sterilization gas is selected from a group consisting of: ethylene oxide, hydrogen peroxide gas, inorganic agents, and organic agents.

6. The self-sealed medical sterilization pouch of claim 4, wherein the second sheet (20) is made of non-tearing film.

7. The self-sealed medical sterilization pouch of claim 4, wherein the second sheet (20) is made of plastic.

8. The self-sealed medical sterilization pouch of claim 1, wherein the two sterile processing indicators (13a and 13b) are sterile ink dots and separately responsive to different sterilizing agents.

9. The self-sealed medical sterilization pouch of claim 1, wherein the second sheet (20) comprises a gap (16) formed at one end of the second sheet.

## Patentansprüche

1. Selbstdichtender medizinischer Sterilisationsbeutel mit einem ersten Blatt (10), einem zweiten Blatt (20), das mit dem ersten Blatt (10) durch eine erste Dichtlinie (22), eine zweite Dichtlinie (23) und eine dritte Dichtlinie (24) verbunden ist, welche an der Kante von drei Seiten des ersten Blatts (10) und des zweiten Blatts (20) angeordnet sind und einen geschlossenen Raum (12) mit einer Öffnung (21) bilden, und wobei der geschlossene Raum (12) mindestens ein zu sterilisierendes medizinisches Instrument (30) fasst, wobei die dritte Dichtlinie (24) die innerste der drei Dichtlinien (22, 23 und 24) ist;
mindestens einen Druckbereich (11), der auf der Oberfläche des ersten Blattes (10) bereitgestellt ist und von der ersten Dichtlinie (22) und der zweiten Dichtlinie (23) eingeschlossen ist, um den geschlossenen Raum (12) sauber zu halten;
eine Klappe (14), die an einem Ende des ersten Blatts (10) bereitgestellt ist und sich auf der Seite der Öffnung (21) befindet, wobei eine Faltlinie (15) zwischen dem geschlossenen Raum (12) und der Klappe (14) vorliegt;
eine trennende Dichtlinie (24a), die in dem geschlossenen Raum (12) nahe an der dritten Dichtlinie (24) bereitgestellt ist;
und mindestens zwei sterile Prozessindikatoren (13a und 13b), die auf der Oberfläche des ersten Blatts (10) bereitgestellt sind,
wobei die trennende Dichtlinie (24a) einen gebogenen Abschnitt (241a), ein erstes kellenförmiges Ende (242a) und ein zweites kellenförmiges Ende (243a) umfasst, wobei das erste kellenförmige Ende (242a) und das zweite kellenförmige Ende (243a) jeweils mit zwei Enden des gebogenen Abschnitts (241a) verbunden sind; und
wobei die mindestens zwei sterilen Prozessindikatoren (13a und 13b) zwischen der dritten Dichtlinie (24) und dem ersten kellenförmigen Ende (242a) bzw. zwischen der dritten Dichtlinie (24) und dem zweiten kellenförmigen Ende (243a) angeordnet sind.

2. Selbstdichtender medizinischer Sterilisationsbeutel nach Anspruch 1, wobei das erste Blatt (10) für Dampf, Wasserdampf, und ein Sterilisationsgas durchlässig ist.

3. Selbstdichtender medizinischer Sterilisationsbeutel nach Anspruch 2, wobei das Sterilisationsgas aus einer Gruppe gewählt ist, die besteht aus: Ethylenoxid, Wasserstoffperoxidgas, inorganische Agenzien, und organische Agenzien.

4. Selbstdichtender medizinischer Sterilisationsbeutel nach Anspruch 1, wobei das zweite Blatt (20) undurchlässig für Dampf, Wasserdampf und ein Sterilisationsgas ist.

5. Selbstdichtender medizinischer Sterilisationsbeutel nach Anspruch 4, wobei das Sterilisationsgas aus einer Gruppe gewählt ist, die besteht aus: Ethylenoxid, Wasserstoffperoxidgas, inorganische Agenzien, und organische Agenzien.

6. Selbstdichtender medizinischer Sterilisationsbeutel nach Anspruch 4, wobei das zweite Blatt (20) aus reißfester Folie hergestellt ist.

7. Selbstdichtender medizinischer Sterilisationsbeutel nach Anspruch 4, wobei das zweite Blatt (20) aus Plastik hergestellt ist.

8. Selbstdichtender medizinischer Sterilisationsbeutel nach Anspruch 1, wobei die beiden sterilen Prozessindikatoren (13a und 13b) sterile Tintenpunkte sind und gesondert auf unterschiedliche Sterilisationsagenzien ansprechen.

9. Selbstdichtender medizinischer Sterilisationsbeutel nach Anspruch 1, wobei das zweite Blatt (20) eine Lücke (16) umfasst, die an einem Ende des zweiten Blatts ausgebildet ist.

## Revendications

1. Sachet de stérilisation médical autocollant comprenant une première feuille (10), une seconde feuille (20) qui est attachée à la première feuille (10) par une première ligne d'étanchéité (22), une deuxième ligne d'étanchéité (23) et une troisième ligne d'étanchéité (24) qui sont placées sur le bord de trois côtés latéraux de la première feuille (10) et de la seconde feuille (20) et forment un espace fermé (12) ayant une ouverture (21), et l'espace fermé (12) logeant au moins un instrument médical (30) à stériliser, la troisième ligne d'étanchéité (24) étant la plus interne des trois lignes d'étanchéité (22, 23 et 24),
au moins un domaine d'impression (11) qui est présent sur la surface de la première feuille (10) et bloqué par la première ligne d'étanchéité (22) et la deuxième ligne d'étanchéité (23) pour maintenir l'espace fermé (12) propre,
une languette (14) qui est présente sur une des extrémités de la première feuille (10) et positionnée du côté de l'ouverture (21), une ligne de pliage (15) étant présente entre l'espace fermé (12) et la languette (14),
une ligne d'étanchéité séparative (24a) qui est présente dans l'espace fermé (12) et proche de la troisième ligne d'étanchéité (24),
et au moins deux indicateurs de stérilisation (13a et 13b) qui sont présents sur la surface de la première feuille (10),
la ligne d'étanchéité séparative (24a) comprenant une partie courbe (241a), une première extrémité en forme de louche (242a) et une seconde extrémité en forme de louche (243a), la première extrémité en forme de louche (242a) et la seconde extrémité en forme de louche (243a) étant respectivement attachées à deux extrémités de la partie courbe (241a) ; et
les au moins deux indicateurs de stérilisation (13a et 13b) étant placés respectivement entre la troisième ligne d'étanchéité (24) et la première extrémité en forme de louche (242a) et entre la troisième ligne d'étanchéité (24) et la seconde extrémité en forme de louche (243a) .

2. Sachet de stérilisation médical autocollant selon la revendication 1, dans lequel la première feuille (10) est perméable à une vapeur, la vapeur d'eau et un gaz de stérilisation.

3. Sachet de stérilisation médical autocollant selon la revendication 2, dans lequel le gaz de stérilisation est choisi dans le groupe constitué par l'oxyde d'éthylène, le peroxyde d'hydrogène gazeux, des agents inorganiques et des agents organiques.

4. Sachet de stérilisation médical autocollant selon la revendication 1, dans lequel la seconde feuille (20) est imperméable à une vapeur, la vapeur d'eau et un gaz de stérilisation.

5. Sachet de stérilisation médical autocollant selon la revendication 4, dans lequel le gaz de stérilisation est choisi dans le groupe constitué par l'oxyde d'éthylène, le peroxyde d'hydrogène gazeux, des agents inorganiques et des agents organiques.

6. Sachet de stérilisation médical autocollant selon la revendication 4, dans lequel la seconde feuille (20) est constituée par un film non déchirable.

7. Sachet de stérilisation médical autocollant selon la revendication 4, dans lequel la seconde feuille (20) est constituée par du plastique.

8. Sachet de stérilisation médical autocollant selon la revendication 1, dans lequel les deux indicateurs de stérilisation stériles (13a et 13b) sont des points d'encre stériles et répondent séparément à différents agents de stérilisation.

9. Sachet de stérilisation médical autocollant selon la revendication 1, dans lequel la seconde feuille (20) comprend un espace (16) formé au niveau d'une extrémité de la seconde feuille.
